# EUROPEAN PATENT SPECIFICATION

(11) **EP 3 217 908 B1**
(45) Date of publication and mention of the grant of the patent: **06.05.2020**
(21) Application number: 15798240.6
(22) Date of filing: 09.11.2015
(51) Int. Cl.: A61B 18/22, A61B 18/12, A61B 18/00, A61B 17/00

(54) **MEDICAL LASER SYSTEM INCLUDING A MOBILE ELECTRONIC DEVICE**
MEDIZINISCHES LASERSYSTEM MIT EINER MOBILEN ELEKTRONISCHEN VORRICHTUNG
SYSTÈME MÉDICAL À LASER COMPRENANT UN DISPOSITIF ÉLECTRONIQUE MOBILE

(30) Priority: 10.11.2014 US 201462077731 P
(43) Date of publication of application: 20.09.2017
(73) Proprietor: biolitec Unternehmensbeteiligungs II AG, 1030 Wien (AT)
(72) Inventor: NEUBERGER, Wolfgang, Dubai (AE); SCHNITKER, Kai, 53332 Bornheim (DE)
(74) Representative: Herrmann, Franz
(86) International application number: PCT/IB2015/058653
(87) International publication number: WO 2016/075613

(56) References cited:
- EP-A1- 2 617 380
- WO-A1-2014/037524
- US-A1- 2013 041 292
- US-A1- 2014 012 242
- US-B1- 8 202 268

## Description

### Background of the Invention

### 2. Field of the invention

The present disclosure relates to medical devices, and more particularly to medical devices delivering electromagnetic radiation, such as laser radiation, microwave radiation or RF radiation.

### 3. Invention Disclosure Statement

Medical devices delivering electromagnetic radiation, such as laser devices, are currently used by physicians in a variety of medical treatments to produce a variety of physical effects such as cutting tissue, closing vessels, liquefying fatty tissue, coagulating blood, destroying calculi, ablating tissue or activating photodynamic drugs. The range of clinical applications is enormous, consequently, more precise and sophisticated medical radiation emitting medical devices are being developed. However, these powerful devices are knowledgeable for their potential hazards, so efforts on protecting patients and personnel against injuries or undesired effects are also a major concern in these improvements. The challenges of today's medical devices are pushing the developments to more sophisticated devices which demand more functions and features, better displays and interfaces. However, the technological advancement frequently leads to medical equipments that are expensive, bulky, and that involve time-consuming training for the users. The German utility model DE 20 2012 005 295U1 by Health & Life Co., Ltd., provides a medical near-field communication system to transmit measured physiological parameters to a smartphone, tablet PC or notebook user who stores and processes the data. However, it does not offer an improvement over the medical equipments themselves. Similarly, the International Publication WO2014111468 by Michael Mankopf et al., provides apparatus for wireless control of a medical apparatus using a remote control but does not enhances the medical apparatuses as in present disclosure.Currently available medical systems often lack user-friendliness and are not designed in a compact easy-to-use device. Thus, there is a need for compact medical treatment systems that improves on the state of the art, to provide an optimal combination of versatility, portability and user-friendliness for existing and future medical treatments. The present invention addresses these needs.

The above information is presented as background information only to assist with an understanding of the present disclosure. No determination has been made, and no assertion is made, as to whether any of the above might be applicable as prior art with regard to the present disclosure.

US 8 202 268 B1 discloses a system without the components being fixed in relation to each other.

### Objectives and Brief Summary of the Invention

While the invention is defined in claim 1, aspects of the present disclosure are to address at least the above-mentioned problems and/or disadvantages and to provide at least the advantages described below.

Accordingly, one advantage of the present disclosure is to provide a compact medical system that combines the complexity of electromagnetic radiation emitting devices for medical applications with the advanced computing capability and connectivity of high-tech mobile electronic devices.

Another advantage is to provide a portable medical system which is capable to be used as a hand-held medical system.

Yet another advantage of the subject systems is that they merge the requirements and features of sophisticated medical devices, which are more regulated as to safety of essential tasks, with the high-tech and rich features of mobile electronic devices.

A further advantage of some embodiments is that they provide medical systems that combine the low to medium volume production of medical devices with the high volume production of mobile electronic devices so as to obtain a cost effective solution that benefits from the mass production scales but takes the safety requirements into account as well.

Briefly stated, present disclosure provides portable medical systems, comprising at least one electromagnetic radiation emitting device for medical applications, at least one mobile electronic device, and at least one attachable/detachable transmission medium. The electromagnetic radiation emitting device comprises a radiation source such as a laser source, a radiofrequency source, or a microwave source and required safety features/-controls. The mobile electronic device has advanced computing capability and connectivity, and is capable of wirelessly accessing a network; such as a smart phone, a tablet personal computer, and the like. The transmission mediums are optical fibers, handpieces, fiber-optic systems, radiofrequency catheters and electrodes, having differently shaped distal tips, differently shaped support tubes, and lens assemblies with different focal distances. This makes system versatile, as it can be easily adapted to a large number of diverse medical applications.

The above and other objects, features and advantages of the present invention herein and/or of the currently preferred embodiments thereof will become more readily apparent from the following description read in conjunction with the accompanying drawings, in which like reference numbers in different drawings designate the same elements.

### Brief Description of Figures

Fig. 1A and 1B illustrate an embodiment of present disclosure, showing a hand-held medical system comprising an electromagnetic radiation emitting device, a mobile electronic device, and a transmission medium.
Fig. 2A and 2B are schematic diagrams of a preferred embodiment of a device including a laser source, a smartphone and fiber-optic laser delivery system.
Fig. 3A and 3B show other embodiments of the compact medical system of present disclosure illustrating different ergonomic designs.

### Detailed Description of Preferred Embodiments

The following description with reference to the accompanying drawings is provided to assist in a comprehensive understanding of various embodiments of the present disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope of the present disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the present disclosure. Accordingly, it should be apparent to those skilled in the art that the following description of various embodiments of the present disclosure is provided for illustration purpose only and not for the purpose of limiting the present disclosure as defined by the appended claims and their equivalents.

It is to be understood that the singular forms "a," "an," and "the" include plural referents unless the context clearly dictates otherwise. Thus, for example, reference to "a radiation source" includes reference to one or more of such sources.

One of the main advantages of present invention is that it merges the requirements and features of sophisticated medical devices, taking into account the strict safety precautions that must be observed while operating an electromagnetic radiation emitting device, with the high-tech, friendly interface, connectivity, and rich features of mobile electronic devices such as smart phones or tablets. Furthermore, the use of these technologies in the novel way of present disclosure provides the advantage of combining the benefits of the low to medium production volumes of medical devices with the high production volume of smart phones or tablets, so as to obtain a cost effective solution that benefits from the mass production scales but takes the safety requirements into account as well.

As described further below, the currently preferred embodiments provide a compact medical system comprising one or more electromagnetic radiation emitting devices for medical applications, at least one mobile electronic device, and at least one attachable/detachable transmission medium. The electromagnetic radiation emitting device comprises safety features/controls that medical devices require for medical applications and at least one radiation source such as a laser source, a radiofrequency source, or a microwave source. In a preferred embodiment, the radiation source is a laser source that operates at one or more laser wavelengths. The medical system comprises at least one electromagnetic radiation emitting source; a mobile electronic device; an attachable/detachable transmission medium; and an apparatus that hold said components fixed in relation to each other.

The medical system of present disclosure employs a mobile electronic device having advanced computing capability and connectivity, and is capable of wirelessly accessing a network. The mobile electronic device of present disclosure comprises at least one of, but it is not limited to, for example, a smart phone, a tablet Personal Computer (PC), a mobile phone, a video phone, an e-book reader, a Personal Digital Assistant (PDA), a Portable Multimedia Player (PMP), a camera, and a wearable device (e.g., a Head-Mounted-Device (HMD) such as electronic glasses, an electronic accessory, and a smart-watch). Those skilled in the art shall understand that the mobile electronic device of present disclosure is not limited to these devices. While the present disclosure can be applied to various mobile electronic devices, it is applied to the mobile electronic device such as a smartphone/tablet by way of the following examples.

In some currently preferred embodiments, the attachable/detachable transmission medium has a distal end and a proximal end, wherein the distal end is near or at a treatment site, and the proximal end is connected to the compact medical system. Attachable/detachable treatment components include transmission mediums with differently shaped distal tips, transmission mediums in differently shaped support tubes, and lens assemblies with different focal distances. This makes system versatile, as it can be easily adapted to a large number of diverse medical applications. Preferably, the transmission medium comprises one or more optical fibers, one or more fiber-optic laser delivery systems, one or more handpieces, one or more optical fibers with differently shaped distal tips, one or more optical fibers in differently shaped support tubes, and lens assemblies with different focal distances, one or more radiofrequency electrodes, one or more radiofrequency catheters, and/or microwave transmission media.

Given that different transmission mediums can be connected to the compact medical system, depending on the type of electromagnetic radiation to be delivered by the system, one advantage of this novel system is that it identifies and confirms that the proper transmission medium is attached to system. The system not only recognizes between transmission mediums involving different electromagnetic radiation sources, but also recognizes among different transmission mediums involving one type of electromagnetic radiation source. In some embodiments, the transmission medium is an optical fiber selected from optical fibers with differently shaped distal tips, different core dimensions, and the system recognizes the type of optical fiber connected to the system by RFID recognition.

In other currently preferred embodiments, the medical system is compact and has an ergonomic design that allows the user to manipulate the device minimizing fatigue and discomfort, resulting in more comfort, higher productivity and less stress. In another embodiment, the medical system is portable, mobile and flexible, and is designed to fit the user's hands so that it can be hand-held. However, it is not necessary to hold the system in the hand during the entire procedure. The system is designed to be held in the hand for example only to set the necessary treatment parameters or device settings, to insert the laser fiber and/or to start the procedure. Then the system can be hanged, for example, on an IV pole, or placed on a supporting surface such as a table. The user need not hold the device in the hand continuously.

In another embodiment, the medical system has an apparatus that holds the electromagnetic radiation emitting device/source, the mobile electronic device and the attachable/detachable transmission medium fixed in relation to each other. Such apparatus has an ergonomic design that allows the user to manipulate the device, minimizing fatigue and discomfort. In other embodiments, the apparatus has an ergonomic design that is portable and mobile.

Another feature of some currently preferred embodiments is that the medical system is a compact system that is battery operated. This feature increases portability and provides more freedom to operate as hand-held portable systems. The electromagnetic radiation emitting device is preferably a battery-driven diode laser source. Laser diodes are chosen such that emitting wavelengths and power output capability meet the needs of pre-established medical treatments. One or more rechargeable batteries can be used to power the laser energy source. Preferably, the batteries are contained in the ergonomic grip. More preferably, a docking/recharging station can be used for enabling rapid recharging of the device.

Another feature of some currently preferred embodiments is that internal cooling means to cool the system are a part of the system. In one embodiment, the compact medical system comprises a diode laser device, as the electromagnetic radiation emitting component, operating at one or more wavelengths which is cooled by a fan that draws air and cools the medical system.

In other currently preferred embodiments, the electromagnetic radiation emitting device comprises a laser source that is triggered by applying an external trigger. In a preferred embodiment, the external trigger is selected, but not limited to, a foot-operated lever, a hand-operated lever, a hard-coded button/key, or a predetermined signal from the mobile electronic device.

In other currently preferred embodiments, the system is operated by flexibly programmable buttons of the mobile electronic device, such as Soft Keys, to invoke any of a number of functions described by the text at that moment shown adjacent to the button on the display. Most preferably, the system is operated by flexibly programmable buttons/keys and by hard-coded buttons/keys. In one example, programmable buttons are used to validate the transmission medium, and hard-coded buttons are used as a safety measure to immediately stop the electromagnetic radiation emitting system.

One of the main advantages of using the mobile electronic device, such as a smartphone, is that then the medical system can use readily available high-tech functions and features of the smartphones, such as the display function, touch-screen function, the flexibly programmable buttons, the hard-coded buttons, the decodification capabilities, the advance computing and connectivity capabilities, reading and/or writing capabilities, wireless technology capabilities for connecting and/or exchanging data over short distances from fixed and mobile devices, security, encryption and identity protection capabilities, and all other capabilities already known. In addition, the smartphone also provides the possibility of being controlled remotely, by a remote control.

Another feature of some currently preferred embodiments is the possibility to easily update the system. This could be done by simply replacing the mobile electronic device by a newer or other type of mobile electronic device, or by enabling a network connection for software updates, retrieval of logging files and/or for future software extensions. The mobile electronic device further comprises programmable software which controls the electromagnetic radiation emitting device of the medical system. This software, also known as mobile electronic device "application", supports reading identification tags, such as a RFID tag, via wireless communication, such as Near Field Communications (NFC).

In all preferred embodiments, the medical systems have safety measures that make these devices suitable for medical treatments; highly diminishing the risks to patients and end-users. Advantages of this system include safety-relevant requirements provided by the radiation emitting device while the mobile electronic device provides more complex features of the system. The built-in electronics generally provide safety features not affected by the mobile electronic device. In other embodiments, the medical system comprises a control system that is further controlled by the mobile electronic device. Yet, in other preferred embodiments, the medical system further comprises an extra control system that controls the safety features of the device, such as a microprocessor, capable for example, of limiting the lasing parameters depending on the desired safety and medical requirements of the application, as well as the specifications of the transmission medium. Yet, in other embodiments, the medical system provides a safe system by restricting the type of transmission medium that can be coupled to the medical system with the aid of the mobile electronic device. By providing this feature, the system only allows proper transmission mediums to be coupled to or used by the system. In the case of laser systems for medical applications, characteristics such as maximum power, pulse frequency, optical fiber type, optical fiber diameter, and optimum wavelength range can and should be matched between laser and optical fiber to avoid harming the patient, and to maximize the therapeutic effect.

In one embodiment, the medical system comprises a laser device with safety features/controls for laser treatments, a smartphone and a fiber-optic laser delivery system. The fiber-optic laser delivery system contains a read-write feature that stores the usage history information of the fiber-optic laser delivery system such as the amount of power transmitted through the delivery system, the use duration, the number of uses, and/or the amount of energy delivered. The smartphone decodes and read this information from the read-write feature, as well as writes in the read-write feature, to update the information on the latest use.

In other currently preferred embodiments, the mobile electronic device is connected to the electromagnetic radiation emitting device of the laser system wirelessly, or through known communications systems that transfers data such as universal serial buses.

Some currently preferred embodiments also provide a medical system for delivering laser light to soft tissue in contact and in non-contact mode in surgical procedures, including endoscopic procedures. In a preferred embodiment, the medical system, comprising a laser source that operates at one or more laser wavelengths and optical fibers, is used for endovenous treatments wherein the optical fiber is inserted inside a vein and delivers laser radiation to the vein walls in order to close or reduce the diameter of the vein.

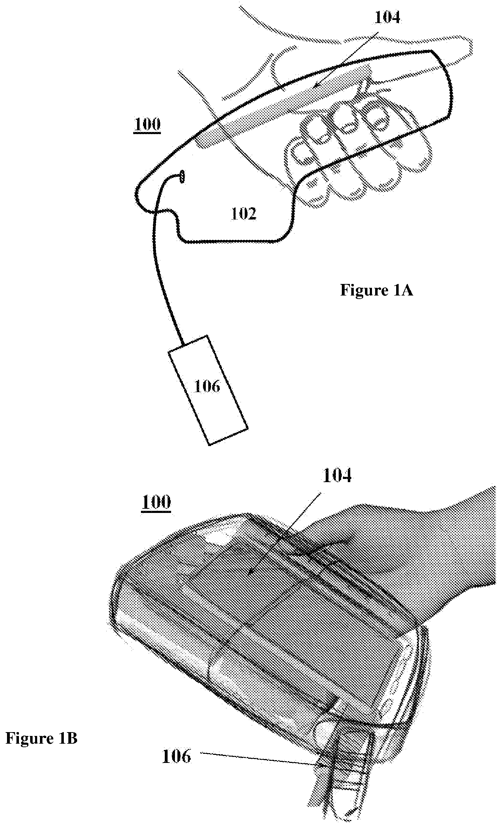

As shown in Figs. 1A and 1B, a first embodiment of a portable medical system is indicated generally by the reference numeral **100**. The portable medical system **100** comprises electromagnetic radiation emitting device **102** that delivers electromagnetic energy for medical applications through attachable/detachable transmission medium **106**, and is controlled by mobile electronic device **104.** Mobile electronic device **104**, such as a tablet personal computer, is attached or detached from electromagnetic radiation emitting device **102** and provides the user interface. The user interacts with the system through mobile electronic device 104. In this embodiment, portable medical system **100** is a hand-held portable system.

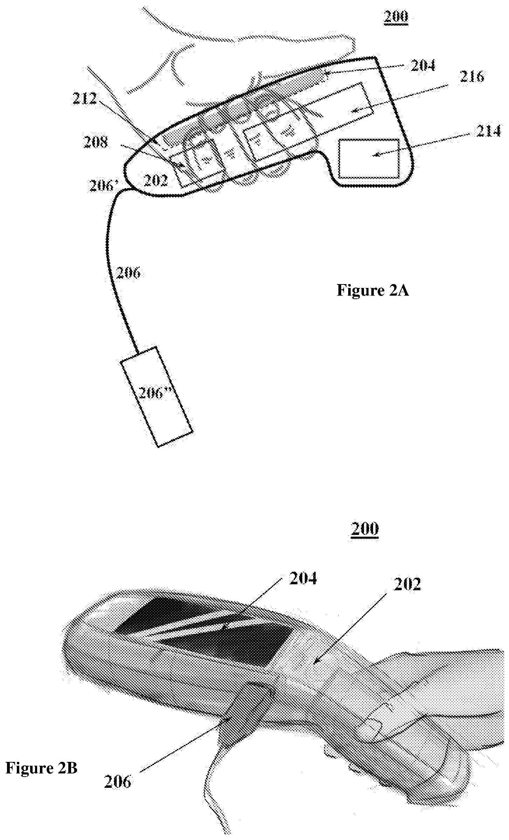

In another preferred embodiment, depicted in Figs. 2A and 2B, a medical system is indicated generally by the reference numeral **200**. Medical system **200** comprises laser radiation device **202** with ergonomic design that generates laser energy with diode laser source **208.** Proximal end **206'** of fiber-optic laser delivery system **206** is optically connected to laser radiation device **202**, and distal end **206"** is placed near or at a treatment site. Laser energy is delivered to the treatment site through fiber-optic laser delivery system **206**. The user places smartphone **204** in smartphone compartment **212,** which connects smartphone **204** to laser radiation device **202**. Once smartphone **204** is in place and connected to laser radiation device **202**, smartphone **204** provides the known smartphone capabilities to medical system **200**. User interacts with medical system through smartphone's **204** interface and controls laser radiation device **202**. Smartphone **204** further recognizes the type of fiber-optic laser delivery system **204** connected to medical system **200** by radiofrequency identification (RFID) recognition. Medical system **200** is further designed to have room within its structure to house power sources **214**, such as batteries. Power sources **214** are preferably rechargeable, and may be recharged for example by mounting laser radiation device **202** on a docking/recharging station (not illustrated). Medical system **200** further comprises fan **216** that draws air and cools the system.

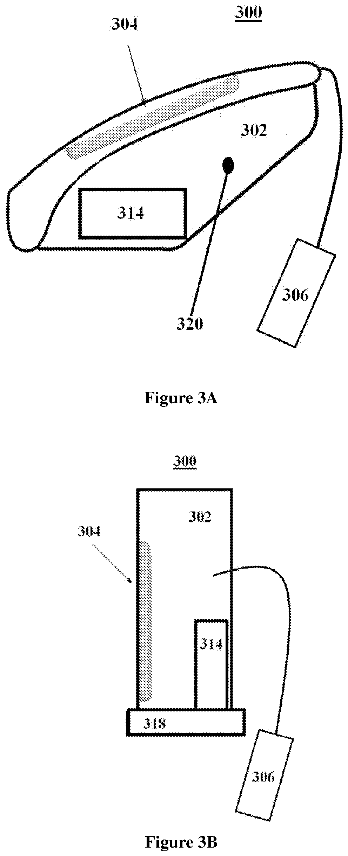

Figs. **3A** and **3B** show other embodiments of the medical system of present disclosure illustrating different ergonomic designs. Fig. 3A shows medical system **300** comprising RF radiation device **302,** radiofrequency electrodes **306** connected to RF radiation device **302,** and tablet **304,** which provides user interface and the central control of the system. Medical system **300** houses power source **314**. Medical system **300** is charged by charging cable connection **320**. Fig. 3B shows another configuration of medical system **300** wherein the system is placed on docking/recharging station **318**, as an alternative option to recharge the system.

A significant advantage of the currently preferred embodiments is that they fulfill the safety requirements of medical devices while at the same time provide a simple and user friendly interface, so as to obtain a cost effective solution that benefits from the mass production scales of smartphones taking into account the safety and efficiency required by medical devices.

The present invention is further illustrated by the following examples, but is not limited thereby.

### Example 1:

In currently preferred embodiments, a medical system comprises a laser emitting device operating at one or more wavelengths, one smartphone, and attachable/detachable optical fibers with different laser emitting tips. The smartphone supports the user interface and enables the user to interact with the system. The smartphone controls and communicates with the laser device through a USB connection. The user selects the desired/required optical fiber and connects it to the medical system. The optical fibers have a RFID chip at the connector which stores basic information of the fiber such as usage restrictions, fiber type, fiber diameter, etc. The optical fiber is optically connected to the laser device through the connector. The laser device is connected to the smartphone via a USB connector. The smartphone uses its decoding and connectivity capabilities to detect the optical fiber, decode the information contained in the RFID chip and determine whether or not the optical fiber is suitable for use with the laser source. The smartphone connects to the RFID via Near Field Communication (NFC), and after fiber verification, the smartphone enables the laser device trough the USB port that connects the smartphone to the laser device. The smartphone not only recognizes the optical fiber, but it is also able to limit the lasing parameters according to pre-programmed standards that assure safety conditions. These standards may be read and decoded by the smartphone, or may be manually entered to the smartphone, via the smartphone interface, which then controls the laser device. After the optical fiber is used and the medical treatment performed, the smartphone writes the RFID chip of the optical fiber and updates the optical fiber's usage information. The smartphone provides a graphical user interface informing the user the status of the system during the procedure, and also allows the surgeon to introduce additional information about the patient, including the option to upload pictures/photos from files and the option to save the GPS positioning data. The camera or beamer of the smartphone also provides the user with a tool for taking pictures and/or projecting images before, during or after a medical procedure. The user either fires the laser by pressing a footswitch or a hand switch, connected to the smartphone via a wireless connection, such as a Bluetooth connection, or by pressing the soft/hard buttons of the smartphone. A hard button in the smartphone is always accessible in case any event requires immediate halt of laser emission.

### Example 2:

A tablet in inserted in and connected to the handheld RF system and takes over control of the RF system. The tablet is connected to the RF source via a wireless connection, and a RF catheter is coupled to the RF source. A small incision is made in the leg of the patient and the RF catheter is inserted into the vein. Using ultrasound guidance, the catheter is positioned in a predetermined section of the vein. Once in proper position, the surgeon uses the tablet interface to start the RF source and deliver radiofrequency energy to the vein wall, causing it to heat, collapse and seal shut.

### Example 3:

It is widely known that different medical treatments involve the delivery of different laser wavelengths, so numerous laser wavelengths are currently used in medical applications. A person skilled in the art would understand that this example is not intended to restrict the disclosure to this specific treatment, but to exemplify one application. A portable laser system, designed to be hold in the hand while the parameters of the treatment are set up, comprises a laser source, a tablet personal computer (PC) and a flexible optical fiber having a radiation emitting tip that emits radiation laterally and annularly with respect to the elongated axis of the optical fiber from its distal tip. The tablet PC is connected to the laser source via a USB connector. The optical fiber is optically coupled to the portable laser system through a standard connector. The optical fiber has a RFID tag that is decoded by the tablet PC. Once the tablet PC decodes and recognizes the optical fiber, it enables the laser source. The tablet PC is connected via a Bluetooth connection to a footswitch. When the surgeon presses the footswitch, the tablet PC fires the laser. The surgeon makes a small incision in the leg of the patient, introduces the optical fiber into the vein and places the optical fiber in a predetermined section of the vein, all under ultrasound guidance. The portable laser system delivers laser light to the vein walls in order to close or reduce the diameter of the vein. While lasing, the portable laser device is hanged or placed in a surface, so the surgeon concentrates in withdrawing the fiber while delivering laser energy to the vein. The tablet PC application offers a friendly interface to the user and provides information useful for the surgeon that performs the treatment such as the battery lifetime, the signal mode, the amount of power and/or energy being delivered, and the status of the laser. It also allows the surgeon to introduce laser parameters such as laser power, energy delivery mode, and time. The surgeon can also take pictures before the treatment is performed, and as soon as the treatment is finished. This same tablet PC is then used to take follow up pictures of the patient.

Having described preferred embodiments of the invention with reference to the accompanying drawings, it is to be understood that the invention is not limited to the precise embodiments, and that various changes and modifications may be effected therein by skilled in the art without departing from the scope of the invention as defined in the appended claims.

## Claims

1. A medical system comprising
at least one electromagnetic radiation emitting device (102, 202, 302);
at least one mobile electronic device (104, 204, 304) to control and communicate with the electromagnetic radiation emitting device (102, 202, 302); and
at least one attachable and detachable transmission medium (106, 206, 306), having a distal end and a proximal end, wherein said distal end is placeable near or at a treatment site; and
an apparatus that holds said electromagnetic radiation emitting device (102, 202, 302), said mobile electronic device (104, 204, 304) and said attachable/detachable transmission medium (106, 206, 306) fixed in relation to each other,
wherein said medical system is portable and mobile; and/or is usable as a hand-held medical system.

2. The system according to claim 1, wherein said apparatus has an ergonomic design.

3. The system according to claim 1 or 2, wherein said at least one electromagnetic radiation emitting device (102, 202, 302) comprises safety features or controls and a source selected from the group consisting of a laser source, a radiofrequency source, and a microwave source.

4. The system according to claim 3, wherein said at least one electromagnetic radiation emitting device (102, 202, 302) comprises safety features or controls and a laser radiation source that operates at one or more laser wavelengths.

5. The system according to any one of claims 1 to 4, wherein the mobile electronic device has computing capability and connectivity and is capable of wirelessly accessing a network.

6. The system according to any one of claims 1 to 5, wherein said mobile electronic device (104, 204, 304) is a device selected from the group consisting of smart phone, a tablet personal computer, a mobile phone, a video phone, an e-book reader, a personal digital assistant, a portable multimedia player, a camera, and a wearable device.

7. The system according to any one of claims 1 to 6, wherein said attachable and detachable transmission medium (106, 206, 306) is selected form the group consisting of at least one optical fiber, optical fibers with differently shaped distal tips, optical fibers in differently shaped support tubes, at least one fiber-optic laser delivery system, lens assemblies with different focal distances, at least one handpiece, at least one radiofrequency electrode, at least one radiofrequency catheter and at least one microwave transmission medium.

8. The system according to any one of claims 1 to 7, utilizing said mobile electronic device capability to decode in order to identify said attachable and detachable transmission medium (106, 206, 306).

9. The system according to any one of claims 1 to 8, utilizing said mobile electronic device NFC capability for reading and/or writing from or to the detachable transmission medium (106, 206, 306).

10. The system according to any one of claims 1 to 9, utilizing said mobile electronic device display and/or touch screen for displaying and/or inputting information.

11. The system according to any one of claims 1 to 10, utilizing said mobile electronic device Bluetooth capability for footswitch or hand switch interface.

12. The system according to any one of claims 1 to 11, utilizing said mobile electronic device camera and/or beamer for taking and/or projecting images before, during or after an intervention.

## Patentansprüche

1. Medizinisches System mit
wenigstens einem elektromagnetische Strahlung emittierenden Gerät (102, 202, 302);
wenigstens einem mobilen elektronischen Gerät (104, 204, 304) zur Steuerung und zur Kommunikation mit dem elektromagnetische Strahlung emittierenden Gerät (102, 202, 302); und
wenigstens einem anbringbaren und entfernbaren Transmissionsmedium (106, 206, 306) mit einem distalen Ende und einem proximalen Ende, wobei das distale Ende in der Nähe oder an einer Behandlungsstelle anordnenbar ist; und
eine Vorrichtung, die das Strahlung emittierende Gerät (102, 202, 302), das mobile elektronische Gerät (104, 204, 304) und das anbringbare/entfernbare Transmissionsmedium (106, 206, 306) in einem festen Verhältnis untereinander hält,
wobei das medizinische System tragbar und mobil ist und/oder als ein in der Hand gehaltenes medizinisches System verwendet wird.

2. System nach Anspruch 1, bei dem die Vorrichtung ergonomisch gestaltet ist.

3. System nach Anspruch 1 oder 2, bei dem das wenigstens eine elektromagnetische Strahlung emittierende Gerät (102, 202, 302) Sicherheitsmerkmale oder -bedienelemente aufweist und eine Quelle, die aus der Gruppe ausgewählt ist, die aus einer Laserquelle, einer Radiofrequenzquelle und einer Mikrowellenquelle besteht.

4. System nach Anspruch 3, bei dem das wenigstens eine elektromagnetische Strahlung emittierende Gerät (102, 202, 302) Sicherheitsmerkmale oder -bedienelemente und eine Laserstrahlungsquelle aufweist, die bei einer oder mehreren Laserwellenlängen arbeitet.

5. System nach einem der Ansprüche 1 bis 4, bei dem das mobile elektronische Gerät Rechenfähigkeiten und Verbindungsfähigkeiten aufweist und in der Lage ist, auf ein Netzwerk drahtlos zuzugreifen.

6. System nach einem der Ansprüche 1 bis 5, bei dem das mobile elektronische Gerät (104, 204, 304) ein Gerät ist, das aus der Gruppe ausgewählt ist, die aus einem Smartphone, einem Tablet-Computer, einem Mobiltelefon, einem Bildtelefon, einem E-Buch-Lesegerät, einem persönlichen digitalen Assistenten, einem tragbaren Multimedia-Abspielgerät, einer Kamera und einem tragbaren Gerät besteht.

7. System nach einem der Ansprüche 1 bis 6, bei dem das anbringbare und entfernbare Transmissionsmedium (106, 206, 306) aus der Gruppe ausgewählt ist, die wenigstens aus einer optischen Faser, optischen Fasern mit verschiedenen gestalteten distalen Spitzen, optischen Fasern in verschieden gestalteten Halteröhren, wenigstens einem faseroptischen Laserzuführsystem, Linsenzusammenstellungen mit verschiedenen fokalen Entfernungen, wenigstens einem Handstück, wenigstens einer Radiofrequenzelektrode, wenigstens einem Radiofrequenzkatheter und wenigstens einem Mikrowellenübertragungsmedium besteht.

8. System nach einem der Ansprüche 1 bis 7, das die Fähigkeit des mobilen elektronischen Geräts zum Decodieren dazu verwendet, das anbringbare und entfernbare Transmissionsmedium (106, 206, 306) zu identifizieren.

9. System nach einem der Ansprüche 1 bis 8, das die NFC-Fähigkeit des mobilen elektronischen Geräts dazu verwendet, vom oder auf das entfernbare Transmissionsmedium (106, 206, 306) zu lesen und/oder zu schreiben.

10. System nach einem der Ansprüche 1 bis 9, das die Anzeige und/oder den berührungsempfindlichen Schirm des mobilen elektronischen Geräts dazu verwendet, Informationen anzuzeigen und/oder einzugeben.

11. System nach einem der Ansprüche 1 bis 10, das die Bluetoothfähigkeit des mobilen elektronischen Geräts für eine Schnittstelle zu einem Fußschalter oder Handschalter verwendet.

12. System nach einem der Ansprüche 1 bis 11, das die Kamera und/oder den Projektor des mobilen elektronischen Geräts dazu verwendet, um Bilder vor, während oder nach einer Behandlung aufzunehmen und/oder zu projizieren.

## Revendications

1. Système médical comprenant
au moins un dispositif d'émission de rayonnement électromagnétique (102, 202, 302) ; au moins un dispositif électronique mobile (104, 204, 304) pour commander et communiquer avec le dispositif d'émission de rayonnement électromagnétique (102, 202, 302) ; et
au moins un support de transmission pouvant être connecté et déconnecté (106, 206, 306) ayant une extrémité distale et une extrémité proximale, dans lequel ladite extrémité distale peut être placée près ou au niveau d'un site de traitement ; et
un appareil qui contient ledit dispositif d'émission de rayonnement électromagnétique (102, 202, 302), ledit dispositif électronique mobile (104, 204, 304) et ledit support de transmission pouvant être connecté/déconnecté (106, 206, 306) fixés les uns par rapport aux autres,
dans lequel ledit système médical est portable et mobile ; et/ou peut être utilisé en tant que système médical de poche.

2. Système selon la revendication 1, dans lequel ledit appareil a un design ergonomique.

3. Système selon la revendication 1 ou 2, dans lequel ledit au moins un dispositif d'émission de rayonnement électromagnétique (102, 202, 302) comprend des fonctions ou commandes de sécurité et une source sélectionnée parmi le groupe constitué d'une source laser, d'une source de radiofréquence et d'une source de micro-ondes.

4. Système selon la revendication 3, dans lequel ledit au moins un dispositif d'émission de rayonnement électromagnétique (102, 202, 302) comprend des fonctions ou commandes de sécurité et une source de rayonnement laser qui opère à une ou plusieurs longueurs d'onde laser.

5. Système selon l'une quelconque des revendications 1 à 4, dans lequel le dispositif électronique mobile a une capacité de calcul et une connectivité et est capable d'accéder sans fil à un réseau.

6. Système selon l'une quelconque des revendications 1 à 5, dans lequel ledit dispositif électronique mobile (104, 204, 304) est un dispositif sélectionné parmi le groupe constitué d'un smartphone, d'un PC tablette, d'un téléphone mobile, d'un visiophone, d'une liseuse, d'un assistant personnel, d'un lecteur multimédia portable, d'une caméra et d'un dispositif portable.

7. Système selon l'une quelconque des revendications 1 à 6, dans lequel ledit support de transmission pouvant être connecté et déconnecté (106, 206, 306) est sélectionné parmi le groupe constitué d'au moins une fibre optique, de fibres optiques avec des pointes distales de forme différente, de fibres optiques dans des tubes de support de forme différente, d'au moins un système de fourniture laser à fibre optique, d'ensembles de lentilles avec différentes distances focales, d'au moins un embout, d'au moins une électrode à radiofréquence, d'au moins un cathéter à radiofréquence et d'au moins un support de transmission à micro-ondes.

8. Système selon l'une quelconque des revendications 1 à 7, utilisant ladite capacité du dispositif électronique mobile à décoder afin d'identifier ledit support de transmission pouvant être connecté et déconnecté (106, 206, 306).

9. Système selon l'une quelconque des revendications 1 à 8, utilisant ladite capacité NFC du dispositif électronique mobile pour lire et/ou écrire depuis ou sur le support de transmission pouvant être déconnecté (106, 206, 306).

10. Système selon l'une quelconque des revendications 1 à 9, utilisant ledit écran et/ou écran tactile du dispositif électronique mobile pour afficher et/ou entrer des informations.

11. Système selon l'une quelconque des revendications 1 à 10, utilisant ladite capacité Bluetooth du dispositif électronique pour une interface à interrupteur au pied ou manuel.

12. Système selon l'une quelconque des revendications 1 à 11, utilisant ladite caméra et/ou ledit projecteur du dispositif électronique mobile pour prendre et/ou projeter des images avant, au cours de ou après une intervention.
